# EUROPEAN PATENT APPLICATION

(11) **EP 2 465 853 A1**
(43) Date of publication of application: **20.06.2012**
(21) Application number: 10194881.8
(22) Date of filing: 14.12.2010
(51) Int. Cl.: C07D 249/04, A61K 31/4192

(54) **Polymorph of rufinamide and process for obtaining it**

(71) Applicant: Laboratorios Lesvi, S.L., 08970 Sant Joan Despi (ES)
(72) Inventor: Sola Carandell, Lluis, 43007 Tarragona (ES); Freixas Pascual, Glòria, 43007 Tarragona (ES); Ceròn Bertran, Jordi, 43007 Tarragona (ES); Dalmases Barjoan, Pere, 08980 Sant Feliu De Llobregat (ES)
(74) Representative: Ponti Sales, Adelaida

(57) **Abstract**

The present invention refers to a new polymorph of Rufinamide, designed as Form R-5, the process for obtaining it, the composition containing it and its use as medicament.

The new polymorph of Rufinamide shows good stability and appropriate physico-chemical properties for its manipulation on industrial scale. Polymorph Form R-5 will be suitable to use as pharmaceutical for the treatment of convulsions, especially for the treatment of epilepsy.

## Description

### FIELD OF THE INVENTION

The present invention refers to a new polymorphic form of Rufinamide, process for obtaining it, and its use in pharmaceutical compositions comprising this polymorphic form.

### BACKGROUND OF THE INVENTION

Rufinamide presents an anticonvulsant activity and it is used to treat epileptic patients with partial and generalized tonic-clonic convulsions as well as various other seizures disorders. Its chemical name is 1-(2,6-difluorobenzyl)-1*H*-1,2,3-triazole-carboxamide and has the structure depicted below:

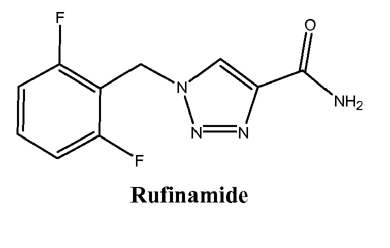

Rufinamide was first disclosed in the American patent US 4,789,680 by Ciba-Geigy. The preparation of Rufinamide, as described in patent US 4,789,680, is depicted in Scheme 1. As seen in Scheeme 1, Rufinamide is prepared from intermediate 1-(2,6-difluorobenzyl)-1*H*-1,2,3-triazole-4-carboxylic acid (1*H*-I) by treatment with thionyl chloride followed by addition of concentrated aqueous ammonia solution. Afterwards, Rufinamide is recrystallised from ethanol. US 4,789,680 gives no indication that such recrystallization is specifically to be applied on particular conditions that might be adopted.

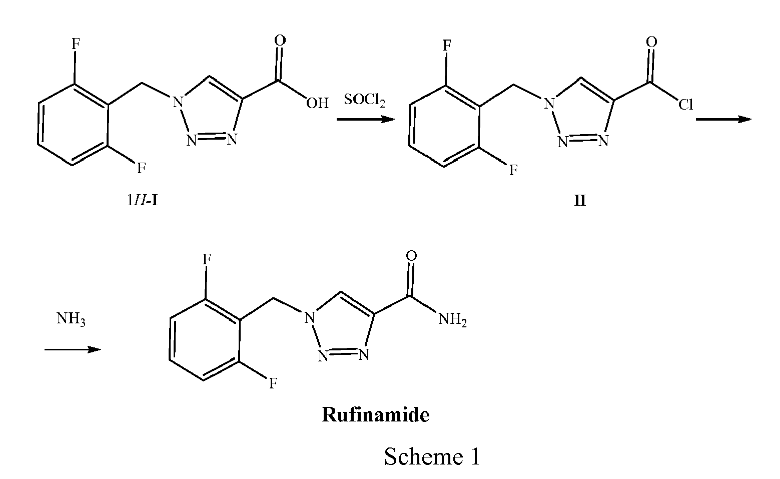

European patents EP0994 863 and EP0994864 disclose polymorphic forms of Rufinamide referred as, crystal modification A, A', B and C. According to the teachings of these patents, crystal modifications A or A' have better thermodynamic stability than crystal modifications B and C. Crystal modification C, the least stable of the three crystal modifications, can only be obtained under very specific conditions.

However, it has been experimentally found that the crystal modification C is rapidly converted into modification B at room temperature within a few weeks. The modification C is also converted into either the modification A or A' or into the modification B, depending on experimental conditions. Moreover, the modification A or A' has a slower dissolution rate in water or in gastric fluid (so-called "slow-release effect").

One of the most desirable properties of a pharmaceutical compound, which can form different polymorphs (that is the case of Rufinamide), is its solubility in aqueous solution, in particular the solubility of said compound in the gastric juices of a patient. Another important property relates to the ease of processing the polymorphic form into pharmaceutical dosages, as the tendency of a powdered or granulated form to flow as well as the surface properties, determine whether the crystals of said polymorphic form will adhere to each other when compacted into a tablet.

The discovery of new polymorphic forms and solvates of a pharmaceutically useful compound provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic.

### BRIEF DESCRIPTION OF THE INVENTION

The Inventors of the present invention have surprisingly found that Rufinamide can further exist in a new polymorphic form, referred herein as R-5. The new polymorphic form, R-5, shows good stability and appropriate physico-chemical properties for its manipulation at industrial scale. As a result of that, the polymorphic form R-5 of the invention will be suitable to be used in pharmaceutical compositions showing advantages over known crystalline forms of Rufinamide.

Thus, a first aspect of the present invention is to provide a new crystalline form of Rufinamide, referred as form R-5 and characterized by data selected from X-ray powder diffraction pattern, DSC thermogram, IR spectrum and TGA thermogram.

The invention also relates to a process for the preparation of form R-5 of Rufinamide, which allows obtaining it in good yields. Therefore, a second aspect of the invention is to provide a process for obtaining said polymorphic form R-5 of Rufinamide.

A third aspect of the invention is directed to the use of the polymorphic form R-5 of Rufinamide for the treatment of convulsions of various origins such as for the treatment of epilepsy.

### DESCRIPTION OF THE FIGURES

Figure 1 shows the X-ray powder diffraction pattern (XRPD) of polymorphic form R-5 of Rufinamide.
Figure 2 shows the differential scanning calorimetry (DSC) and the thermogravimetric analysis (TGA) of polymorphic form R-5 of Rufinamide.
Figure 3 shows the infrared (IR) spectrum of polymorphic form R-5 of Rufinamide.

### DETAILED DESCRIPTION OF THE INVENTION

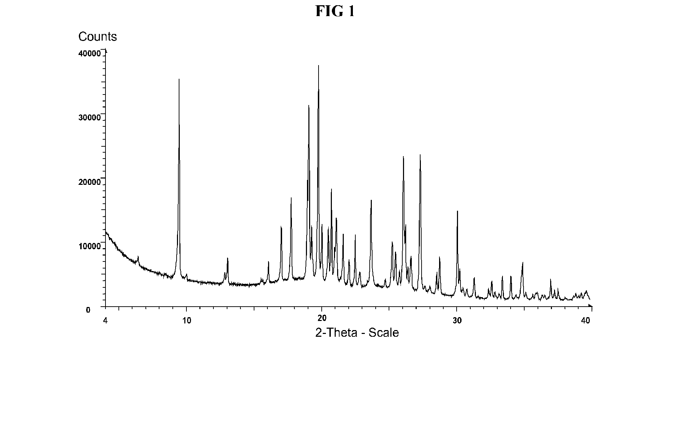

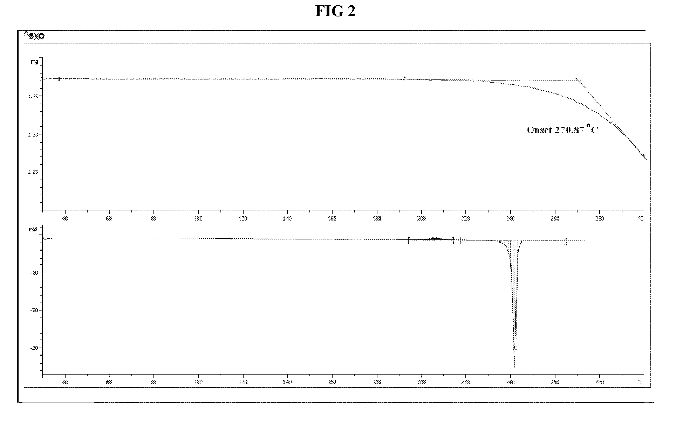

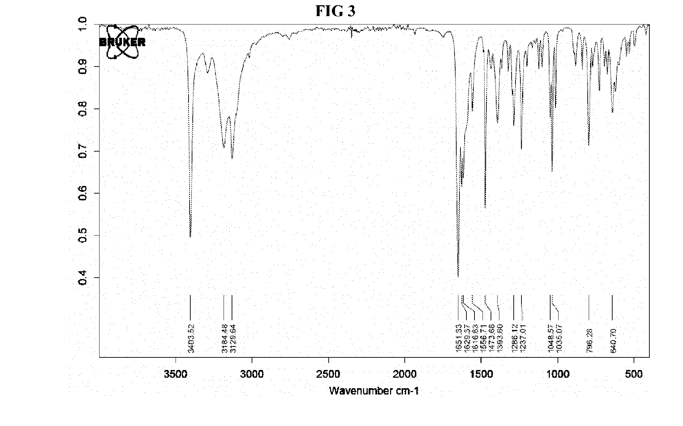

According to the first aspect, the invention provides a new polymorphic form of Rufinamide, referred as form R-5, which is characterized by its X-ray powder diffraction pattern (Figure 1), its DSC thermogram and thermogravimetric analyses (Figure 2) and its infrared (IR) spectra (Figure 3).

XRD patterns was obtained using an analytical X'Pert PRO MPD alpha 1 powder diffractometer, equipped with a CuKα source (λ = 1.54056 Å) and a X'Celerator Detector, which operates at 40 kV and 40 mA. Each sample was scanned between 4 and 40° in 2θ, with a step size of 0.016° and a scan rate of 40 s/step. The PXRD of crystalline phase R-5 of Rufinamide are depicted in Table 1 and figure 1.

**Table 1**

| Angle 2θ¹ (°) | Relative Intensity % | | (cont.) Angle 2θ¹ (°) | (cont.) Relative Intensity % |
|---|---|---|---|---|
| 6,4 | 20,1 | | 21,0 | 24,1 |
| 9,4 | 85,1 | | 21,1 | 36,3 |
| 9,9 | 12,8 | | 21,6 | 29,5 |
| 12,8 | 13,2 | | 22,0 | 18,7 |
| 13,0 | 19,6 | | 22,5 | 29,2 |
| 15,5 | 10,8 | | 22,8 | 13,5 |
| 15,7 | 10,4 | | 23,7 | 43,8 |
| 16,0 | 17,9 | | 24,7 | 10,4 |
| 17,0 | 32,1 | | 25,2 | 26,4 |
| 17,7 | 44,6 | | 25,5 | 22,0 |
| 18,9 | 52,4 | | 25,8 | 14,1 |
| 19,0 | 83,4 | | 26,1 | 62,1 |
| 19,2 | 32,5 | | 26,2 | 33,9 |
| 19,7 | 100,0 | | 26,6 | 20,3 |
| 20,0 | 33,6 | | 26,4 | 16,4 |
| 20,5 | 31,7 | | 27,3 | 62,8 |
| 20,7 | 48,4 | | 27,6 | 7,8 |
| ¹The 2θ values were obtained using copper radiation (Cu_{Kα1} 1.54060Å) | | | | |

Differential scanning calorimetry was carried out by means of a Mettler-Toledo DSC-822e calorimeter using aluminum crucibles, and a heating rate of 10°C/min, from 30° up to 300 °C. The measurements were carried out in a dry nitrogen atmosphere using a 50 ml/min flow rate, The calorimeter was calibrated with indium of 99.99% purity.

The differential scanning calorimetry graph showed a weak exothermic peak with a maximum at 204-206°C, a sharp endothermic peak with an onset at 239°C and a maximum at 240°C (208J/g).

Thermogravimetric analysis (TGA) was performed on a Mettler-Toledo SDTA851e thermobalance. Experimental conditions: alumina crucibles of 40 µl volume, atmosphere of dry nitrogen with 80 ml/min flow rate, heating rate of 10°C/min starting at 30° up to 300 °C. In the thermogravimetric analysis no weight loss was observed before decomposition occurred. Weight loss due to decomposition was only observed at temperatures over 220°C, see Figure 2.

FTIR spectrum was recorded on a Bruker Alpha spectrometer, equipped with a Bruker Diamond single reflection ATR system, a mid-infrared source as the excitation source and a DTGS detector. The spectrum were acquired in 32 scans with a resolution of 4 cm⁻¹ in the range of 4000-650 cm⁻¹. The IR spectrum obtained is characterised by the following bands: 3404, 3185, 3130, 1651, 1629, 1617, 1557, 1473, 1394, 1286, 1237, 1049, 1035, 796 and 640 (cm⁻¹).

Advantageously, the inventors have found that polymorphic form R-5 does not change by grinding, in the precedence of solvent or without solvent.

Grinding experiments were performed in a Retsch MM400 Ball Mill using the following methodology: samples of 30 mg of phase R-5 of Rufinamide were grinded at a frequency of 20 s⁻¹ for 15 min, with a drop of solvent and without solvent. The results obtained are shown in Table 2.

**Table 2**

| Initial solid | Solvent | Solids after grinding¹ |
|---|---|---|
| R-5 | - | R-5 |
| R-5 | Water | R-5 |
| R-5 | Ethanol | R-5 |
| R-5 | Isopropanol | R-5 |
| R-5 | 1-Propanol | R-5 |
| R-5 | 2-Butanol | R-5 |
| R-5 | Dichloromethane | R-5 |
| R-5 | 1,2-Dichloroethane | R-5 |
| R-5 | Tetrahydrofurane | R-5 |
| R-5 | Methyl ethyl ketone | R-5 |
| R-5 | Toluene | R-5 |
| ¹) The solid phase was determined by PXRD. | | |

Further advantages of polymorphic form R-5 arose from the significant good thermal stability that has shown over time. No changes in polymorphic form was seen when form R-5 of rufinamide underwent accelerated degradation. Thermal stability experiments were performed in a Binder Vacuum oven VD53 using the following methodology. Samples of from R-5 of Rufinamide were kept under vacuum, at 40 °C for different periods of times (at 1 mbar), see table 3 below. There were no significant differences in the PXRD patterns of the solids obtained after the thermal treatment.

**Table 3**

| Entry | Initial solid | Temperature | Time | Solids after thermal treatment¹ |
|---|---|---|---|---|
| 1 | R-5 | 40°C | 1 hour | R-5 |
| 3 | R-5 | | 24 hours | R-5 |
| 6 | R-5 | | 24 hours | R-5 |
| 9 | R-5 | | 24 hours | R-5 |
| 12 | R-5 | | 7 hours | R-5 |
| (¹-) The solid phase was determined by PXRD. | | | | |

Further experiments were carried out to study the effect of humidity on the stability of new form R-5. The relative humidity (RH) was set and controlled using a

Projekt Messtechnik Modular Humidity Generator using the following methodology: samples of 10 mg of phase R-5 of Rufinamide were kept at 70% RH and at 50°C for 24 hours and at 90% RH at 30°C for 67 hours.

**Table 4**

| Entry | Initial solid | Relative humidity | Temperature | Time | Solids after humidity experiments¹ |
|---|---|---|---|---|---|
| 1 | R-5 | 70% | 50°C | 24 hours | R-5 |
| 2 | R-5 | 90% | 30°C | 67 hours | R-5 |
| (¹-) The solid phase was determined by PXRD. | | | | | |

In another aspect, the invention provides a process for obtaining polymorphic form R-5 of Rufinamide. Thus, the present invention provides a process for preparing polymorphic form R-5 of Rufinamide comprising:
(a) providing a solution of 1-(2,6-difluorobenzyl)-1H-1,2,3-triazole-4-carbonyl chloride in anhydrous tetrahydrofuran;
(b) generating ammonia *in-situ* while maintaining the pH of the mixture below 9 to obtain a precipitate; and
(c) recovering the crystalline form R-5 of Rufinamide,

1-(2,6-difluorobenzyl)-1H-1,2,3-triazole-4-carbonyl chloride used in step (a) may be prepared from 1-(2,6-difluorobenzyl)-1*H*-1,2,3-triazole-4-carboxylic acid substantially free of 3*H*-I isomer as described in European patent application EP10161546.6 still not published and those description is incorporated here by reference, specially pages 4 to 8.

In the present invention, the expression "generating ammonia *in-situ"* means that no addition of ammonia is carried out and that the ammonia which allows the reaction with 1-(2,6-difluorobenzyl)-1H-1,2,3-triazole-4-carbonyl chloride is generated or built in the same reaction media. The formation of ammonia is achieved by combining an ammonium salt with a base with a pKb lower than of ammonia. Surprisingly, the authors of the present invention have found that the reaction of 1-(2,6-difluorobenzyl)-1H-1,2,3-triazole-4-carbonyl chloride with the ammonia generated *in-situ* in the reaction media allows to obtain a new polymorphic form R-5, which has advantage compared to the known polymorphic forms of Rufinamide. See the experimental data included above. For example, ammonia may be formed *in-situ* when using ammonium chloride and sodium bicarbonate.

Step (b) may be performed in the absence or in the presence of water. Another object of the present invention is a pharmaceutical composition comprising a therapeutically effective amount of polymorphic form R-5 of Rufinamide defined above, together with an appropriate amount of pharmaceutically acceptable excipients and carriers.

The above compositions can be administered by any suitable route. As stated above, the polymorphic form R-5 of Rufinamide for the manufacture of a medicament for the treatment of convulsions of various origins, for example for the treatment of epilepsy is also encompassed by the present invention.

The polymorphic form R-5 of Rufinamide is also useful for obtaining other polymorphic forms of rufinamide. In particular, a new polymorphic form, referred as R-4, was also identified. Polymorphic form R-4 was characterised by means of PXRD, DSC and FTIR. The characteristic peaks of the PXRD of crystalline phase R-4 of Rufinamide are depicted in Table 5.

**Table 5**

| Angle 2θ¹ (°) | Relative Intensity % | | Angle 2θ¹ (°) | Relative Intensity % |
|---|---|---|---|---|
| 17,2 | 9,3 | | 24,8 | 11,8 |
| 17,3 | 6,7 | | 25,3 | 7,3 |
| 18,1 | 30,0 | | 25,8 | 8,3 |
| 18,4 | 7,3 | | 27,4 | 5,7 |
| 18,9 | 100,0 | | 27,5 | 5,9 |
| 19,3 | 15,1 | | 32,1 | 21,3 |
| 19,6 | 19,5 | | 33,5 | 6,4 |
| 20,7 | 5,6 | | 36,6 | 16,1 |
| 24,6 | 60,3 | | | |
| ¹The 2θ values were obtained using copper radiation (Cu_{Kα1} 1.54060Å) | | | | |

Characterisation by differential scanning calorimeter DSC (10 °C/min) showed a sharp endothermic peak with an onset at 240 °C and a maximum at 240 °C (206 J/g). Measurements carried out by TGA (10 °C/min) showed no weight loss before decomposition occurred. Weight loss due to decomposition occurred at temperatures over 220 °C. The IR spectrum obtained for form R-4 is characterised by the following bands: 3408, 3179, 3086, 1627, 1595, 1560, 1472, 1397, 1282, 1234, 1037 and 797 cm⁻¹.

The present invention is further illustrated by the following examples. They should in no case be interpreted as a limitation of the scope of the invention as defined in the claims.

### EXAMPLES

### Example 1. Preparation of 1-(2,6-difluorobenzyl)-1H-1,2,3-triazole-4-carboxamide (Rufinamide polymorphic form R-5)

1.9 ml (22.4 mmol) of oxalyl chloride were added drop wise, to a suspension of 5 g (20.9 mmol) of 1-(2,6-difluorobenzyl)-1H-1,2,3-triazole-4-carboxylic acid in 12,5 ml of anhydrous THF, at 20-25 °C and under nitrogen atmosphere. Once the addition of oxalyl chloride was completed, the mixture was stirred at 20-25 °C for 4 h. Afterwards, the resulting mixture was concentrated under vacuum to dryness, in order to eliminate HCl and unreacted oxalyl chloride. The obtained residue was dissolved in 30 ml of fresh anhydrous THF and treated with 4.0 g (75.2 mmol) of ammonium chloride and 5.26 g (62.7 mmol) of sodium bicarbonate over 16 h at 20-25 °C. The resultant suspension was treated with 30 ml of water. The solid was filtrated and washed with 30 ml of a saturated aqueous sodium bicarbonate solution and twice with 30 ml of water. The solid was dried in a vacuum drier at 50 °C to yield 2.78 g (55,8 %) of 1-(2,6-difluorobenzyl)-1H-1,2,3-triazole-4-carboxamide as Form R-5.

### Example 2. Preparation of 1-(2,6-difluorobenzyl)-1H-1,2,3-triazole-4-carboxamide (Rufinamide polymorphic form R-5)

3.74 ml (45.65 mmol) of oxalyl chloride were added drop wise to a suspension of 10 g (41.8 mmol) of 1-(2,6-difluorobenzyl)-1H-1,2,3-triazole-4-carboxylic acid in 25 ml of anhydrous THF, at 20-25°C and under nitrogen atmosphere. When the addition of oxalyl chloride was concluded, the mixture was stirred at 20-25°C for 4 h. Afterwards, the resulting mixture was diluted with 50 ml of anhydrous THF, followed by the distillation of 15 ml at 35-40°C under vacuum, in order to remove the remaining HC1. The reaction was cooled to 20-25°C and 8 g (149.57 mmol) of ammonium chloride were added followed by addition of 20 ml of 8% aqueous solution of sodium bicarbonate. After further addition of 8.8 g (104.97 mmol) of sodium bicarbonate, the mixture was left under powerful stirring over 4 h at 20-25°C. To conclude with, the suspension was filtered and washed twice with 50 ml of a saturated aqueous sodium bicarbonate solution and twice with 50 ml of water The solid was dried on a vacuum drier at 50°C to yield 5.6 g (56.2 %) of 1-(2,6-difluorobenzyl)-1H-1,2,3-triazole-4-carboxamide as Form R-5 .

### Example 3. Preparation of 1-(2,6-difluorobenzyl)-1H-1,2,3-triazole-4-carboxamide (Rufinamide polymorphic form R-5)

75.4 ml of Oxalyl chloride (919.8 mmol) were added at 25°C to a stirred suspension of (2,6-difluorobenzyl)-1H-1,2,3-triazole-4-carboxylic acid (200.0 g, 836.2 mmol) in THF (500 ml) and DMF (0.5 ml). The stirring was maintained for 4 hr at 25 °C until the solid dissolved. Afterwards, 1000 ml of THF were added and reduced pressure (200-250 mbar) was applied leading to the distillation of 300 ml of THF and to the maintenance of the internal temperature at about 25-35 °C. After that, the mixture was slightly cool to 25°C and 160 g of ammonium chloride were added in portions followed by slow addition of 400 ml of an 8% NaHCO3 solution and 176 g of solid NaHCO3 (vigorous stirring was also needed to prevent frothing). The heterogeneous mixture was stirred for 2 hr, filtered and the solid re-suspended in 1 L water. The solid was filtered and dried (at 200 mbar/ 50°C) yielding 130 g (65.3%) of 1-(2,6-difluorobenzyl)-1H-1,2,3-triazole-4-carboxamide, as a white solid in form R5.

### Example 4. Preparation of 1-(2,6-difluorobenzyl)-1H-1,2,3-triazole-4-carboxamide (Rufinamide polymorphic form R-4)

A suspension of Rufinamide form R-5 (110 mg, 0.46 mmol) in a 1:1 mixture of methanol and water (35 mL) was heated at reflux for one hour. The solution obtained was cooled down to 20 °C in four hours without stirring. The resulting suspension was filtered off and the white solid obtained was dried under vacuum (1 mbar) at 40 °C for 4 hours (80 mg, 73% yield). The solid was analyzed by PXRD and corresponded to phase R-4.

### Example 5. Preparation of 1-(2,6-difluorobenzyl)-1H-1,2,3-triazole-4-carboxamide (Rufinamide polymorphic form R-4)

A suspension of Rufinamide, polymorphic form A, (100 mg, 0.42 mmol) in a 1:1 mixture of methanol and water (37 mL) was heated at reflux for one hour. The solution obtained was cooled down to room temperature in ten hours without stirring. The resulting suspension was filtered off and the white solid obtained was dried under vacuum (1 mbar) at 40 °C for 1 hour (75 mg, 75% yield). The solid was analyzed by PXRD and corresponded to phase R-4.

## Claims

1. Polymorph of Rufinamide, referred as Form R-5, wherein said form R-5 has a X-ray powder diffraction pattern spectrum comprising peaks at about: 9.4, 17.0, 17.7, 18.9, 19.0, 19.2, 19.7, 20.0, 20.5, 20.7, 21.1, 23.7, 26.1, 26.2, 27.3 degrees 20 (±0.2)

2. Polymorph of Rufinamide according to claim 1, having a powder X-ray diffraction pattern including the following peaks at about:
| Angle 2θ (°) (±0.2) | | (cont.) Angle 2θ (°) (±0.2) |
|---|---|---|
| 6,4 | | 20,7 |
| 9,4 | | 21,0 |
| 13,0 | | 21,1 |
| 16,0 | | 21,6 |
| 17,0 | | 22,0 |
| 17,7 | | 22,5 |
| 18,9 | | 23,7 |
| 19,0 | | 25,2 |
| 19,2 | | 26,2 |
| 19,7 | | 26,6 |
| 20,0 | | 27,3 |
| 20,5 | | |

3. Polymorph of Rufinamide according to claim 1, having a X-ray powder diffraction pattern spectrum in accordance with Figure 1.

4. Polymorph of Rufinamide according to claim 1, having in the thermogram obtained by DSC a weak exothermic peak with a maximum at 204-206°C, a sharp endothermic peak with an onset at 239°C and a maximum at 240°C in accordance with Figure 2.

5. Polymorph of Rufinamide according to claim 1, having the following absorptions in the IR spectrum: 3404, 3185, 3130, 1651, 1629, 1617, 1557, 1473, 1394, 1286, 1237, 1049, 1035, 796 and 640 (cm⁻¹), in accordance with Figure 3.

6. Process for obtaining the polymorph of Rufinamide, designated as Form R-5, as defined in any of previous claims 1 to 5, comprising:
a) providing a solution of 1-(2,6-difluorobenzyl)-1H-1,2,3-triazole-4-carbonyl chloride in an aprotic organic solvent;
b) generating ammonia *in-situ* while maintaining the pH of the mixture below 9 to obtain a precipitate; and
c) recovering the crystalline form.

7. Process according to claim 6, wherein the generation of ammonia *in-situ* is carried out combining an ammonium salt with a base with the proviso that said base has a pKb lower than the ammonia pKb.

8. Process according to claim 7, wherein said ammonium salt is ammonium chloride and said base is sodium bicarbonate.

9. A pharmaceutical composition comprising a therapeutically effective amount of the polymorph of Rufinamide, referred as Form R-5, as defined in claims 1 to 5, together with the appropriate amount of pharmaceutically acceptable excipients or carriers.

10. Polymorph of Rufinamide, designated as Form R-5, according to any of claims 1 to 5 for use as a medicament.

11. Polymorph of Rufinamide, designated as Form R-5, according to any of claims 1 to 5 for the treatment of convulsions.

12. Polymorph of Rufinamide, designated as Form R-5, according to claim 11 for the treatment of epilepsy.
